(19) Europäisches Patentamt
European Patent Office
Office européen des brevets

(11) **EP 3 536 359 A1**

(12) **EUROPÄISCHE PATENTANMELDUNG**

(43) Veröffentlichungstag:
**11.09.2019  Patentblatt 2019/37**

(51) Int Cl.:
*A61M 1/06* (2006.01)     *G01W 1/14* (2006.01)

(21) Anmeldenummer: **18160184.0**

(22) Anmeldetag: **06.03.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Medela Holding AG**
**6340 Baar (CH)**

(72) Erfinder:
• **THÜRING, Martin**
  **5643 Sins (CH)**
• **HÖNER, Sebastian**
  **8800 Thalwil (CH)**

(74) Vertreter: **Clerc, Natalia**
**Isler & Pedrazzini AG**
**Giesshübelstrasse 45**
**Postfach 1772**
**8027 Zürich (CH)**

(54) **BRUSTHAUBENEINHEIT**

(57)   Eine Brusthaubeneinheit einer Brustpumpe zum Abpumpen von menschlicher Muttermilch weist eine Brusthaube (1) zur Auflage an eine Mutterbrust, einen Milchsammelbehälter (4) zur Aufnahme von abgepumpter Muttermilch, einen Brusthaubenadapter (2) zur Verbindung der Brusthaube (1) mit dem Milchsammelbehälter (4) sowie mindestens einen Sensor (6, 7) zur Detektion der Muttermilch auf. Ein Milchkanal erstreckt sich von der Brusthaube durch den Brusthaubenadapter in den Milchsammelbehälter. Im Milchkanal ist ein Siphon angeordnet und der mindestens eine Sensor (6, 7) zur Detektion der Muttermilch ist in Strömungsrichtung der Muttermilch nach dem Siphon angeordnet. Die Brusthaubeneinheit ermöglicht eine optimale Messung von Eigenschaften der abgepumpten Muttermilch.

FIG. 4

EP 3 536 359 A1

**Beschreibung**

TECHNISCHES GEBIET

Die vorliegende Erfindung betrifft eine Brusthaubeneinheit einer Brustpumpe zum Abpumpen von menschlicher Muttermilch sowie eine Sensoreinheit einer Brustpumpe und ein Verfahren zum Bestimmen von mindestens einer Eigenschaft von menschlicher Muttermilch während des Abpumpens.

STAND DER TECHNIK

Zum Abpumpen von menschlicher Muttermilch wird auf die Mutterbrust eine Brusthaube aufgesetzt, welche mit einer Vakuumpumpe verbunden ist, um die Brust mit einem variierenden Unterdruck zu beaufschlagen. Der Unterdruck variiert vorzugsweise zyklisch oder entsprechend einer vorgegebenen bzw. empirisch evaluierten Saugkurve.

Die abgepumpte Milch gelangt durch einen Milchkanal von der Brusthaube in einen Milchsammelbehälter. Um das Totvolumen beim Abpumpen möglichst klein zu halten, ist ein Milchkanal von der Brusthaube zum Milchsammelbehälter üblicherweise mit einem Rückschlagventil versehen, welches sich für den Einlass der abgepumpten Milch in den Milchsammelbehälter öffnet. Dieses Rückschlagventil ist vorzugsweise an einem Brusthaubenadapter angeordnet, welcher die Brusthaube mit dem Milchsammelbehälter und üblicherweise auch die Brusthaube mit der Vakuumpumpe oder mit einem zur Vakuumpumpe führenden Vakuumschlauch verbindet. Das Rückschlagventil kann auf einem Ventilkopf angeordnet sein, welcher fest oder lösbar mit dem Brusthaubenadapter, auch Kopplungsteil genannt, verbunden ist. Brusthaube und Adapter sind üblicherweise zwei separate Bauteile. Sie können jedoch auch gemeinsam einstückig ausgebildet sein.

Eine derartige Vorrichtung ist beispielsweise aus WO 2014/161099 A1 bekannt. Die dort beschriebene Brusthaubeneinheit weist einen Durchflusssensor auf, welcher die Veränderung einer Ventilklappe des Rückschlagventils detektiert, so dass Rückschlüsse auf den Durchfluss der Milch von der Brusthaube in den Milchsammelbehälter gezogen werden können.

US 2016/0220743 A1 offenbart einen Adapter mit Sensoren zur Detektion der einzelnen Tropfen der abgepumpten Milch. Die Sensoren sind in Fliessrichtung der Milch vor dem Rückschlagventil angeordnet.

US 2015/0283311 A1 beschreibt verschiedene Sensoren, welche im Hals oder im Boden der Milchflasche angeordnet sind.

US 2017/0021068 A1 zeigt einen Fluidsensor, welcher mit dem Milchsammelbehälter verbunden ist.

Messungen von im Milchfluss angeordneten Sensoren sind oft zu ungenau, da die Milch je nach Mutter und Abpumpphase nur tropfenweise oder üppiger fliesst. Zudem ist die Milch mit unterschiedlichen Mengen an Luft vermischt, was ebenfalls die Genauigkeit der Messung einer Milchdurchflussmenge beeinträchtigt.

Sensoren, welche die bereits im Milchsammelbehälter aufgenommene Milchmenge messen, sind üblicherweise zur Messung von geringen Milchmengen, z.B. von Mengen bis zu 30 ml, nicht geeignet.

Ein weiterer Faktor, welcher genaue Messungen des Milchflusses bzw. der abgepumpten Milchmenge verhindert, ist die Medientrennmembran. Eine Medientrennmembran wird verwendet, um die Brustpumpe vor Verunreinigungen schützen. Sie trennt den Bereich, in welchem das Vakuum erzeugt wird, vom Bereich, in welchem die Milch fliesst, wobei sie das von der Brustpumpe erzeugte Vakuum in den Innenraum der Brusthaube überträgt. Insbesondere Sensoren, welche sich auf Klappen oder Rückschlagventilen befinden, werden von diesen Medientrennmembranen beeinflusst, da das Verhalten des Rückschlagventils von der Bewegung der Medientrennmembran beeinflusst wird.

Es sind ferner Vorrichtungen zur Messung von Urinmengen bekannt, welche eine Siphonanordnung verwenden. Derartige Vorrichtungen sind in DE 41 06 995 A1, DE 41 14 933 A1, US 3 641 818, US 3 919 455, US 5 656 027, GB 2 031 158 und WO 2010/149708 A1 beschrieben.

DARSTELLUNG DER ERFINDUNG

Es ist eine Aufgabe der Erfindung, eine Brusthaubeneinheit einer Brustpumpe zum Abpumpen von menschlicher Muttermilch zu schaffen, welche eine Optimierung der Messung der abgepumpten Milch ermöglicht.

Diese Aufgabe wird durch eine Brusthaubeneinheit mit den Merkmalen des Patentanspruchs 1, durch eine Sensoreinheit einer Brustpumpe gemäss Anspruch 15 sowie durch ein Verfahren zum Bestimmen von mindestens einer Eigenschaft von menschlicher Muttermilch gemäss Anspruch 16 gelöst.

Die erfindungsgemässe Brusthaubeneinheit einer Brustpumpe zum Abpumpen von menschlicher Muttermilch weist eine Brusthaube zur Auflage an eine Mutterbrust, einen Milchsammelbehälter zur Aufnahme von abgepumpter Muttermilch, einen Brusthaubenadapter zur Verbindung der Brusthaube mit dem Milchsammelbehälter sowie mindestens einen Sensor zur Detektion der Muttermilch auf. Ein Milchkanal erstreckt sich von der Brusthaube durch den Brusthaubenadapter in den Milchsammelbehälter. Der Milchkanal definiert eine Strömungsrichtung der Muttermilch. Erfindungsgemäss ist im Milchkanal ein Siphon angeordnet und der mindestens eine Sensor zur Detektion der Muttermilch ist in Strömungsrichtung der Muttermilch nach dem Siphon angeordnet.

Ein Siphon im Sinne dieser Anmeldung umfasst auch Schleifen, Loopings und andere Geometrien, welche zu einer blockweisen, d.h. stossweisen, Entleerung eines Abschnitts des Milchkanals führen.

Die Siphonanordnung bietet dank ihrem Staubereich eine Beruhigung der unregelmässig einströmenden Milch. Die Siphonanordnung hat zudem den Vorteil, dass sich im Staubereich lediglich die Milch sammelt und sich somit das in anderen Systemen bei der Messung störend auswirkende Luft-Milchgemisch aufgetrennt wird.

Des Weiteren ist vorteilhaft, dass einzelne Milchpakete den nachfolgenden mindestens einen Sensor blockweise passieren. Dadurch ist eine klare Trennschicht zwischen Luft und Milch erkennbar und detektierbar. Das Milchpaket wird auch Milchvolumen oder Milchblock genannt.

Die Detektion der Trennschicht Luft/Milch am Anfang des den Sensor passierenden Milchpakets sowie die Detektion der Trennschicht Milch/Luft am Schluss des den Sensor passierenden Milchpakets und die Auswertung der dadurch erhaltenen Signale in Funktion der Zeit geben Auskunft über die Milchflussmenge und Milchflussrate während einer bestimmten Zeitspanne. Die Gesamtzahl der Milchpakete gibt Auskunft über die Milchmenge. Die Anzahl Milchpakete während einer bestimmten Zeitspanne gibt Auskunft über die abgepumpte Milchmenge während dieser bestimmten Zeit. Es lassen sich somit die Milchmenge und die Durchflussrate während der gesamten Pumpdauer bestimmen wie auch die Milchmenge und Durchflussrate während einzelner Pumpphasen.

Das Fliessverhalten der Milch lässt sich dank der klaren Trennung von Milch und Luft ebenfalls detektieren. Dieses Fliessverhalten gibt zum Beispiel Auskunft über die Viskosität und somit der Zusammensetzung der Milch. Es lassen sich somit Aussagen zum Makronährstoffgehalt der Milch machen, beispielsweise zum Fettgehalt, zu Proteinen und zur Laktose und/oder zu Mischformen davon.

Vorzugsweise ist ein Ventil vorhanden, welches den Milchkanal während des Abpumpens der Muttermilch schliesst und öffnet. Vorzugsweise ist der Siphon in Strömungsrichtung der Muttermilch nach dem Ventil angeordnet.

Diese Brusthaubeneinheit ermöglicht eine Messung der Milchmenge, welche nicht durch den sich ändernden angelegten Unterdruck bzw. durch das angelegte Vakuum beeinträchtigt wird. Das Ventil, welches vorzugsweise ein Einwegventil bzw. ein Rückschlagventil ist, trennt den Abpumpbereich vom Messbereich.

Auch eine allfällig vorhandene Medientrennmembran beeinflusst die Messung somit nicht, da diese lediglich den Abpumpbereich beeinflusst.

Je nach Art der zu messenden Eigenschaft der Milch ist ein Sensor vorhanden oder es sind zwei oder mehrere Sensoren notwendig. Sind zwei oder mehr Sensoren vorhanden, so sind sie in Strömungsrichtung vorzugsweise hintereinander angeordnet.

In einer bevorzugten Ausführungsform weist die Brusthaubeneinheit ein Signalauswertemodul zur Auswertung von Signalen des mindestens einen Sensors auf. Das Signalauswertemodul liefert einen Verlauf der Signale in Funktion der Zeit, wodurch mindestens eine der folgenden Grössen bestimmbar ist: Durchflussmenge, Durchflussrate, Durchflussgeschwindigkeit, Makronährstoffe, Fettgehalt. Das Signalauswertemodul kann im Bereich der Brusthaube, des Brusthaubenadapters, des Milchsammelbehälters oder eines anderen in diesem Bereich befindlichen Bauteils angeordnet sein. Es kann jedoch auch beabstandet zu diesen Bauteilen angeordnet sein, beispielsweise innerhalb eines Gehäuses, welches auch das Brustpumpenaggregat und/oder die Steuerung der Brustpumpe beinhaltet.

In einer bevorzugten Ausführungsform weist der Milchkanal zwischen Ventil und Siphon eine Erweiterung auf. Diese Erweiterung dient zur Beruhigung der durch das Ventil einströmenden Milch, d.h. zur ersten Trennung des Luft-Milchgemischs und zur Vermeidung von Wirbeln. Vorzugsweise ist die Erweiterung ein sich in Strömungsrichtung verjüngender Trichter.

Der Milchkanal weist vorzugsweise über die Länge des Siphons einen gleichbleibenden Durchmesser auf.

Anstelle eines einzigen Lumens des Milchkanals lassen sich auch zwei oder mehr Lumen verwenden, d.h. die Milch kann auch aufgeteilt durch verschiedene Lumen fliessen. Dies ist beispielsweise dann vorteilhaft, wenn in den einzelnen Lumen unterschiedliche Sensoren angeordnet sind, welche unterschiedliche Eigenschaften der Muttermilch detektieren. Dadurch beeinflussen sich die Messungen nicht gegenseitig.

Der Sensor und der Siphon können im Bereich der Brusthaube, des Brusthaubenadapters oder des Milchsammelbehälters angeordnet sein. In bevorzugten Ausführungsformen ist jedoch eine Sensoreinheit vorhanden, welche vorzugsweise zwischen dem Brusthaubenadapter und dem Milchsammelbehälter angeordnet ist, wobei die Sensoreinheit den Siphon des Milchkanals und den mindestens einen Sensor aufweist. Die Sensoreinheit ist vorzugsweise ein separates Bauteil, welches vorzugsweise zerstörungsfrei lösbar mit dem Brusthaubenadapter und dem Milchsammelbehälter verbindbar ist. In anderen Ausführungsformen ist es jedoch mit einem Teil oder als ganzes Bauteil fest mit dem Brusthaubenadapter und/oder dem Milchsammelbehälter verbunden.

Der Milchkanal kann mindestens im Bereich des Siphons durch einen Schlauch oder eine Röhre gebildet sein.

In bevorzugten Ausführungsformen weist die Sensoreinheit jedoch einen Grundkörper auf mit einer Oberfläche und einer diese Oberfläche dicht verschliessenden Abdeckung. Mindestens der Siphon des Milchkanals ist dabei durch eine Nut gebildet, welche in der Oberfläche und/oder in der Abdeckung ausgebildet ist und welche durch das Zusammenwirken der Oberfläche mit der Abdeckung bis auf einen Einlass und einen Auslass dicht verschlossen ist. Derartige Anordnungen lassen sich optimal reinigen, da insbesondere der Siphonbereich über seine gesamte Länge einfach zugänglich ist.

In bevorzugten Ausführungsformen ist die Oberfläche eine radial nach aussen gerichtete Oberfläche des Grundkörpers. Die Abdeckung ist in diesem Fall vorzugsweise ein Deckel, welcher sich auf den Mantel des Grundkörpers auflegen lässt, oder eine Hülse, welche über den Grundkörper gestülpt werden kann.

In anderen bevorzugten Ausführungsformen weist die Sensoreinheit einen nach oben offenen Innenraum auf, in welchem die Oberfläche in einer Wellenform ausgebildet ist. Die Abdeckung ist in diesem Fall vorzugsweise als wellenförmiges Gegenstück ausgebildet.

Die Sensoreinheit lässt sich äusserst kompakt und robust gestalten, wenn der Grundkörper einen Hohlraum zur Aufnahme einer Elektronikeinheit aufweist. Vorzugsweise weist die Elektronikeinheit das Signalauswertemodul auf. Die Elektronikeinheit ist vorzugsweise wasserdicht geschützt, so dass der Grundkörper mit Wasser gespült bzw. im Waschbecken gewaschen werden kann, um den Milchkanal und insbesondere den Siphon zu reinigen.

Vorzugsweise ist die Sensoreinheit im Wesentlichen rotationssymmetrisch ausgebildet. Vorzugsweise ist sie kreiszylinderförmig oder kegelstumpfförmig. Dadurch lässt sie sich fluchtend mit den übrigen Bauteilen der Brusthaubeneinheit ausbilden und sie lässt sich mit diesen über Gewinde- oder Bajonettverbindungen koppeln.

Der mindestens eine Sensor ist in bevorzugten Ausführungsformen mindestens ein optischer Sensor. Es lassen sich jedoch auch andere Arten von Sensoren verwenden, wie z.B. kapazitive Sensoren, kalorimetrische Sensoren, elektrochemische oder induktive Sensoren, Ultraschallsensoren, Temperatursensoren, Sensoren zur Messung des Widerstandes, Impedanzmesser, Laufzeitmesser, Radar und optische Sensoren wie beispielsweise CCD-Kameras. In bevorzugten Ausführungsformen ist mehr als ein Sensor vorhanden. Vorzugsweise sind unterschiedliche Arten von Sensoren vorhanden. Ist ein Lagesensor vorhanden, welcher die Drehposition der Brusthaubeneinheit im Raum bestimmt, so lassen sich gemessene Werte nach Massgabe der gemessenen Lage korrigieren.

Die erfindungsgemässe Sensoreinheit ist Teil einer Brustpumpe zum Abpumpen von menschlicher Muttermilch, wobei die Brustpumpe eine Brusthaube zur Auflage an eine Mutterbrust, einen Milchsammelbehälter zur Aufnahme von abgepumpter Muttermilch, einen Milchkanal, welcher sich von der Brusthaube zum Milchsammelbehälter erstreckt, sowie mindestens einen Sensor zur Detektion der Muttermilch aufweist, wobei der Milchkanal eine Strömungsrichtung der Muttermilch definiert. Die Sensoreinheit weist einen Teil des Milchkanals auf, wobei dieser Teil des Milchkanals einen Siphon ausbildet. Die Sensoreinheit weist in Strömungsrichtung der Muttermilch nach dem Siphon den mindestens einen Sensor zur Detektion der Muttermilch auf. Vorzugsweise ist ein Ventil vorhanden, welches den Milchkanal während des Abpumpens schliesst und öffnet.

Diese Sensoreinheit ist vorzugsweise im Bereich der Brusthaube und des Milchsammelbehälters angeordnet. Liegen jedoch Brusthaube und Milchsammelbehälter weit auseinander, ist sie vorzugsweise entweder im Bereich der Brusthaube oder im Bereich des Milchsammelbehälters angeordnet. Sie kann jedoch auch in einem Bereich dazwischen, also beabstandet zu Brusthaube und Milchsammelbehälter angeordnet sein, beispielsweise im Bereich des Brustpumpengehäuses des Pumpaggregats.

Das erfindungsgemässe Verfahren zur Bestimmung mindestens einer Eigenschaft von menschlicher Muttermilch erfolgt während eines Abpumpens der menschlichen Muttermilch, wobei die Muttermilch durch Anlegen eines sich ändernden Unterdrucks abgepumpt wird. Das Verfahren weist folgende Schritte auf, welche nacheinander, jedoch nicht zwingend unmittelbar aufeinanderfolgend ausgeführt werden:

- Sammeln der Muttermilch in einem Staubereich eines Siphonbereichs,
- blockweises Entleeren des Staubereichs über eine Siphonschleife des Siphonbereichs unter Bildung eines Muttermilchpakets,
- Weiterleiten des Muttermilchpakets zu mindestens einem Sensor,
- Detektion des blockweise weitergeleiteten Muttermilchpakets mittels des mindestens eines Sensors und
- Auswertung von Signalen des Sensors in Funktion der Zeit.

Vorzugsweise wird die Muttermilch zuerst durch ein Ventil geleitet, insbesondere ein Rückschlagventil und erst dann wird sie im Staubereich des Siphonbereichs gesammelt.

Weitere Ausführungsformen sind in den abhängigen Ansprüchen angegeben.

KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

Figur 1          eine perspektivische Darstellung einer ersten Ausführungsform der erfindungsgemässen Brusthaubeneinheit;

Figur 2          einen Längsschnitt durch die Brusthaubeneinheit gemäss Figur 1;

Figur 3          einen Teilschnitt durch eine Sensoreinheit der Brusthaubeneinheit gemäss Figur 1;

Figur 4          einen weiteren Teilschnitt durch die Sensoreinheit der Brusthaubeneinheit gemäss Figur 1;

Figur 5          eine perspektivische Darstellung einer Elektronikeinheit der Sensoreinheit gemäss Figur 1;

Figur 6a bis Figur 6j          schematische Darstellungen eines Siphons einer erfindungsgemässen Brusthaubeneinheit;

Figur 7a          eine graphische Darstellung der Signale von zwei in Strömungsrichtung nacheinander angeordneten Sensoren in Funktion der Zeit;

Figur 7b          eine graphische Darstellung der Signale eines Sensors in Funktion der Zeit;

Figur 8          eine perspektivische Explosionsdarstellung eines Grundkörpers und einer Abdeckung der Sensoreinheit in einer zweiten Ausführungsform;

Figur 9          eine perspektivische Explosionsdarstellung eines Grundkörpers und einer Abdeckung der Sensoreinheit in einer dritten Ausführungsform;

Figur 10          eine perspektivische Explosionsdarstellung eines Grundkörpers und einer Abdeckung der Sensoreinheit in einer vierten Ausführungsform;

Figur 11          eine perspektivische Explosionsdarstellung eines Grundkörpers und einer Abdeckung der Sensoreinheit in einer fünften Ausführungsform;

Figur 12          eine perspektivische Explosionsdarstellung eines Grundkörpers und einer Abdeckung der Sensoreinheit gemäss den Figuren 1 bis 5;

Figur 13          eine perspektivische Explosionsdarstellung eines Grundkörpers und einer Abdeckung der Sensoreinheit in einer siebten Ausführungsform und

Figur 14          einen Längsschnitt durch den Grundkörper mit aufgesetzter Abdeckung gemäss Figur 13.

## BESCHREIBUNG BEVORZUGTER AUSFÜHRUNGSFORMEN

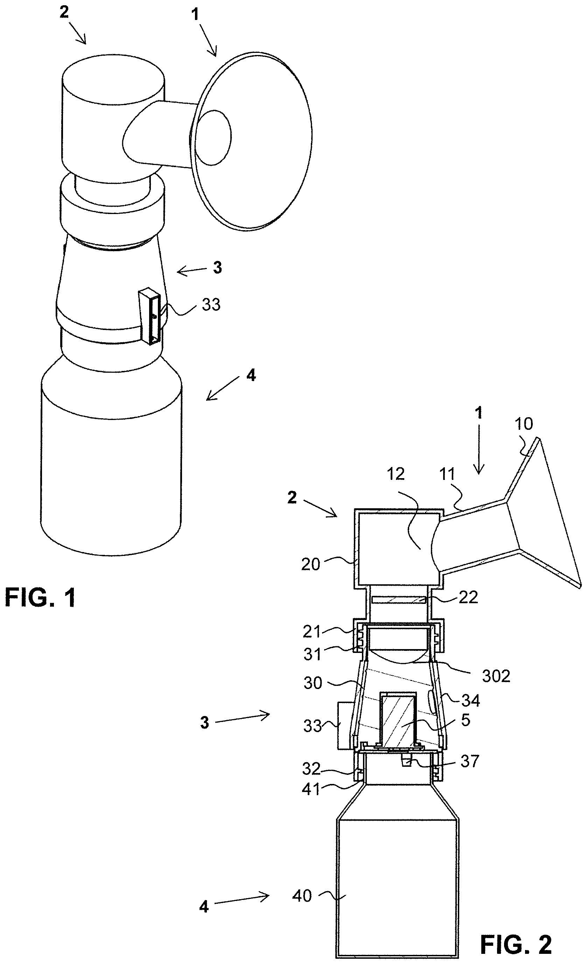

Die Figuren 1 bis 5 zeigen eine erste bevorzugte Ausführungsform der erfindungsgemässen Brusthaubeneinheit. Sie weist, wie in den Figuren 1 und 2 gut erkennbar ist, eine Brusthaube 1, einen Brusthaubenadapter 2, eine Sensoreinheit 3 und einen Milchsammelbehälter 4 auf.

Die Brusthaube 1 weist vorzugsweise einen trichterförmigen Schild 10 zur dichtenden Auflage auf eine Mutterbrust und einen daran anschliessenden rohrförmigen Stutzen 11 auf. Schild 10 und Stutzen 11 sind vorzugsweise gemeinsam einstückig ausgebildet. Sie können aber auch als getrennte ein- oder mehrstückige Bauteile ausgeführt sein. Derartige Brusthauben 1 in unterschiedlichsten Ausführungsformen sind im Stand der Technik hinlänglich bekannt.

Der Brusthaubenadapter 2 ist in diesem Beispiel einstückig mit der Brusthaube 1 ausgebildet. Vorzugsweise ist er jedoch ein separates Bauteil, welches mit der Brusthaube 1 lösbar verbindbar ist.

Er weist vorzugsweise einen Grundkörper 20 mit einem hier nicht sichtbaren Anschluss zur Verbindung mit einer Vakuumquelle auf. Der Anschluss ist je nach Ausführungsform ein Verbindungselement zur Verbindung mit einem Saugschlauch, dessen zweites Ende mit einem Gehäuse eines Pumpaggregats der Brustpumpe verbunden ist. Das Pumpaggregat der Brustpumpe kann jedoch auch direkt am Brusthaubenadapter 2 angeordnet sein. Das Pumpaggregat kann manuell betrieben sein. Vorzugsweise ist es jedoch ein elektromotorisch angetriebenes Pumpaggregat. All diese Varianten sind aus dem Stand der Technik hinlänglich bekannt.

Im Brusthaubenadapter 2 ist je nach Ausführungsform eine Medientrennmembran zwischen Anschluss zur Vakuumquelle und Brusthaube 1 angeordnet. Derartige Medientrennmembranen sind ebenfalls bestens bekannt und sie sind deshalb hier nicht mehr dargestellt.

Im Brusthaubenadapter 2 ist ferner ein Ventil 22 angeordnet. Dieses Ventil 22 ist vorzugsweise ein Einwegventil, insbesondere ein Rückschlagventil. In bevorzugten Ausführungsformen ist es eine einseitig angelenkte Klappe, ein Entenschnabelventil oder ein zentrisch aufgehängtes Ventil. Das Ventil 22 unterteilt den inneren Bereich der Brusthaubeneinheit in einen Abpumpbereich 12 und in einen Messbereich. Im Abpumpbereich 12 wird das sich ändernde Vakuum an die Mutterbrust angelegt. Der Messbereich ist nicht direkt diesen Druckänderungen ausgesetzt. Der Messbereich wird weiter unten genauer beschrieben.

Der Brusthaubenadapter 2 weist ferner einen Anschlussstutzen 21 zur Verbindung mit einem weiteren Bauteil, hier der Sensoreinheit 3, auf. Die Verbindung ist vorzugsweise zwecks Reinigung der einzelnen Bauteile zerstörungsfrei lösbar und somit auch wieder neu erstellbar. Die Verbindung erfolgt vorzugsweise über ein Gewinde oder einen Bajonettverschluss. Die Sensoreinheit 3 weist hierfür ein oberes Anschlussteil 31 auf.

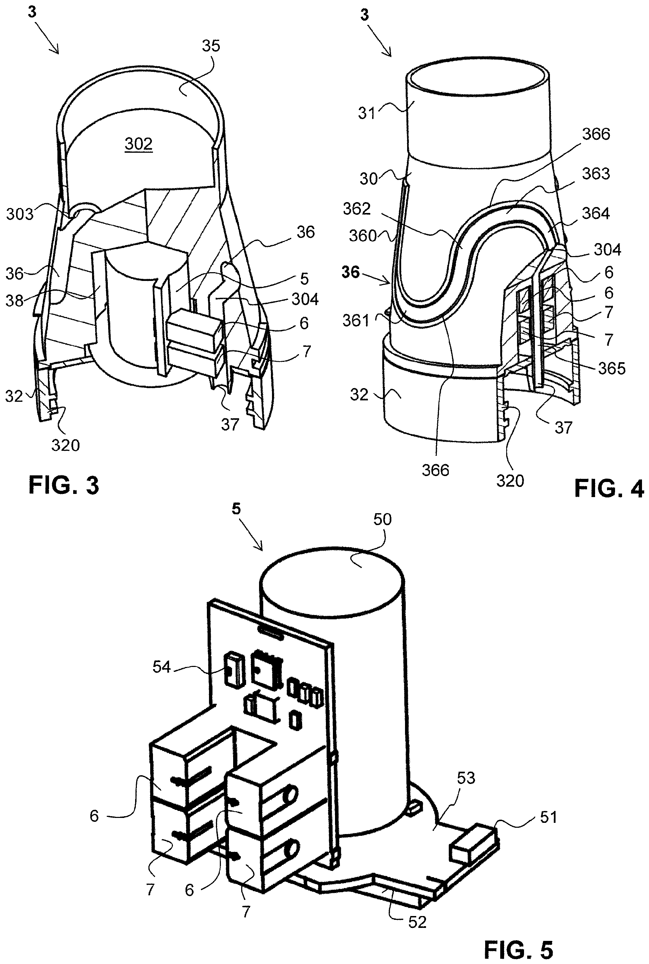

Die Sensoreinheit 3 ist vorzugsweise auch zerstörungsfrei lösbar mit dem Milchsammelbehälter 4 verbunden. Auch hier erfolgt die Verbindung vorzugsweise über ein Gewinde oder über einen Bajonettverschluss. Das Gewinde an einem unteren Anschlussteil 32 der Sensoreinheit 3 ist in den Figuren 3 und 4 mit dem Bezugszeichen 320 versehen. Dieses umgreift einen Hals 41 des Milchsammelbehälters 4, wie in Figur 2 erkennbar ist. Der Grundkörper des Milchsammelbehälters 4 ist mit dem Bezugszeichen 40 versehen.

Vorzugsweise sind die beschriebenen Bauteile im Wesentlichen rotationssymmetrisch ausgebildet. Die Sensoreinheit 3 weist vorzugsweise einen Grundkörper 30 auf, welcher im Wesentlichen die Form eines Kegelstumpfes oder eines Kreiszylinders aufweist.

Dieser Grundkörper 30 ist mindestens über einen Teil seines Mantels, in diesem Beispiel über seinen gesamten Umfang, von einer Abdeckung 34 überdeckt. Dies ist in Figur 2 gut erkennbar. Die Abdeckung 34 dichtet den Grundkörper 30 flüssigkeits- und luftdicht ab. Vorzugsweise lässt sich die Abdeckung 34 zwecks Reinigung der Bauteile zerstörungsfrei vom Grundkörper 30 lösen und wieder montieren.

Der Grundkörper 30 der Sensoreinheit 3 weist vorzugsweise einen zentralen Hohlraum 38 auf, welcher vorzugsweise mindestens flüssigkeitsdicht nach aussen dicht verschlossen ist. Dies ist in den Figuren 2 bis 4 gut erkennbar. In diesem Hohlraum 38 ist eine Elektronikeinheit 5 angeordnet. Diese Elektronikeinheit 5 ist in Figur 5 gut erkennbar. Sie weist eine Leiterplatte 53 mit einer Batterie 50 oder einen anderen Energiespeicher auf. Auf der Leiterplatte 53 ist ferner ein Signalauswertemodul, hier in Form eines Mikrocontrollers 52, angeordnet.

Vorzugsweise wird die Vorrichtung auf einen Energiesparmodus geschaltet, sobald die Sensoreinheit 3 vom Brusthaubenadapter 2 und/oder vom Milchsammelbehälter 4 gelöst wird. Vorzugsweise ist hierfür ein Reedschalter 51 vorhanden, welcher die Stromversorgung trennt bzw. abschaltet, sobald die Abdeckung 34 vom Grundkörper 30 entfernt wird. Ein Halter für einen Reedschalter-Magneten ist in den Figur 1 und 2 mit dem Bezugszeichen 33 versehen.

Ferner weist die Elektronikeinheit 5 vorzugsweise einen TOF Sensor (time-of-flight Sensor) auf, welcher hier nicht sichtbar ist. Er befindet sich vorzugsweise auf der Unterseite der Elektronikeinheit und ist zum Innenraum des Milchsammelbehälters 4 hin gerichtet. Mittels dieses TOF- Sensors lässt sich der Füllstand des Milchsammelbehälters 4 kontinuierlich messen. Vorzugsweise wird diese Messung mit einer Messung des Durchflusses, z.B. der Durchflussrate, des Durchflussvolumens oder der Durchflussmenge, kombiniert.

Mit der Leiterplatte 53 sind ferner Sensoren verbunden. In diesem Beispiel sind eine erste Lichtschranke 6 und eine zweite Lichtschranke 7 vorhanden sowie ein Beschleunigungssensor 54. Mittels dieser oder anderen Sensoren werden vorzugsweise die genannten Eigenschaften der Muttermilch detektiert, insbesondere gemessen.

Ein Milchkanal erstreckt sich durch die gesamte Brusthaubeneinheit. Der Kanal erstreckt sich von der Brusthaube 1, in welche Milch von der Mutterbrust abgepumpt wird, bis zum Innenraum des Grundkörpers 40 des Milchsammelbehälters 4. Der Kanal ist somit durch die in der Brusthaubeneinheit miteinander verbundenen Hohlräume gebildet und er führt durch das Ventil 22. Im Bereich der Sensoreinheit 3 führt der Milchkanal, wie in den Figuren 3 und 4 gut erkennbar ist, durch einen breiten Einlass 35 in eine Trichterstruktur mit einer Schrägfläche 302, welche eine kleine periphere Einlassöffnung 303 aufweist. Diese Einlassöffnung 303 führt zu einem Milchkanalabschnitt 36, welcher sich im Wesentlichen auf der Aussenseite des Grundkörpers 30 befindet und somit als offene Rinne ausgebildet ist. Er weist zwei Schleifen auf und bildet einen Siphon. Er ist vorzugsweise über seine gesamte Länge auf beiden Seiten mit je einer Dichtlippe 366 versehen. Die oben erwähnte Abdeckung 34 überdeckt mindestens diesen Milchkanalabschnitt 36, so dass er mit Ausnahme der Einlassöffnung und einer Auslassöffnung 304 luft- und flüssigkeitsdicht verschlossen ist.

Der Milchkanalabschnitt 36 verläuft, wie in den Figuren 3 und 4 erkennbar ist, von der Einlassöffnung 303 im Innern des Grundkörpers 30 zur Mantelfläche und in einen ersten Kanalbereich 360, welcher im bestimmungsgemässen Gebrauchszustand der Brustpumpeneinheit nach unten verläuft. Der erste Kanalbereich 360 geht in einen ersten Siphonbogen 361 über, welcher in einen nach oben gerichteten zweiten Kanalbereich 362 mündet. Dem zweiten Kanalbereich 362 schliesst ein zweiter Siphonbogen 363 an, an welchen ein wiederum nach unten gerichteter dritter Kanalbereich 364 anschliesst. Der dritte Kanalbereich 364 geht über eine Auslassöffnung 304 in einen im Innern des Grundkörpers 30 verlaufenden vierten Kanalbereich 365 über, welcher in einem nach unten in den Hohlraum des Milchsammelbehälters mündenden Auslass 37 endet.

Wie in den Figuren 3 und 4 erkennbar ist, sind die erste und die zweite Lichtschranke 6, 7 entlang des vierten Kanalbereichs 365 angeordnet. Sie ragen vorzugsweise in den vierten Kanalbereich 365 hinein oder enden bündig mit der Wandung des vierten Kanalbereichs 365. Andere Anordnungen sind möglich. Die erste Lichtschranke 6 befindet sich in Strömungsrichtung der Milch, definiert durch Einlass 35 und Auslass 37, vor der zweiten Lichtschranke 7. Der Bereich um die Lichtschranken 6, 7 ist selbstverständlich luft- und flüssigkeitsdicht versiegelt. Vorzugsweise weist der Milchkanalabschnitt 36 mindestens in den Bereichen des ersten bis dritten Kanalbereichs 360, 362, 364 und der zwei Siphonbögen 361, 363 einen gleichbleibenden Querschnitt auf.

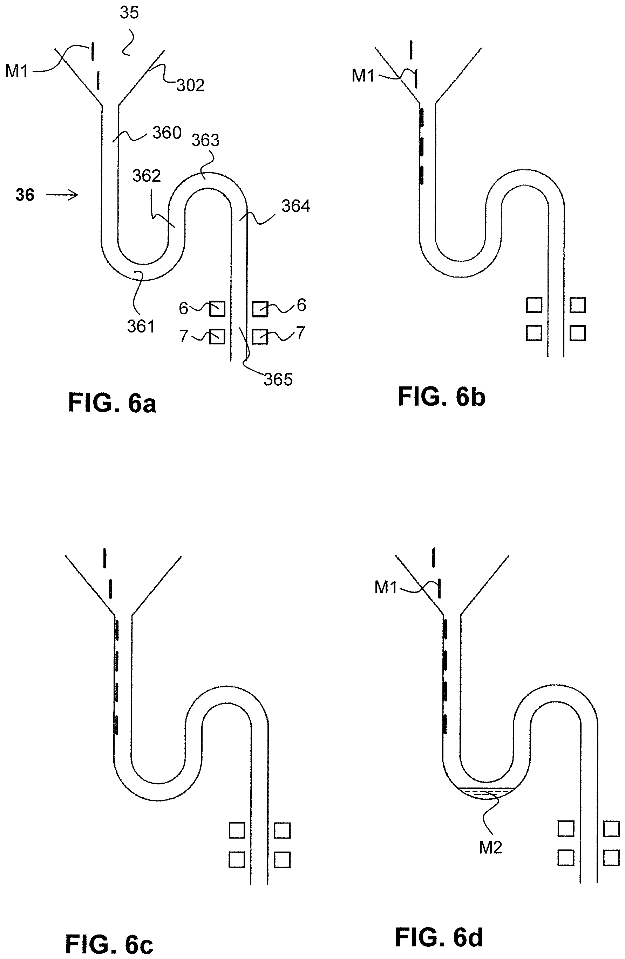

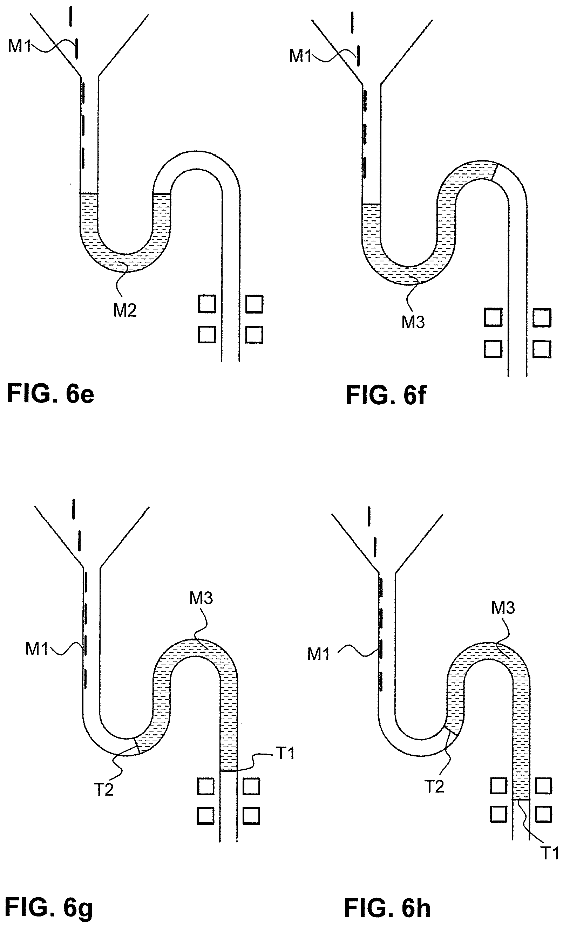

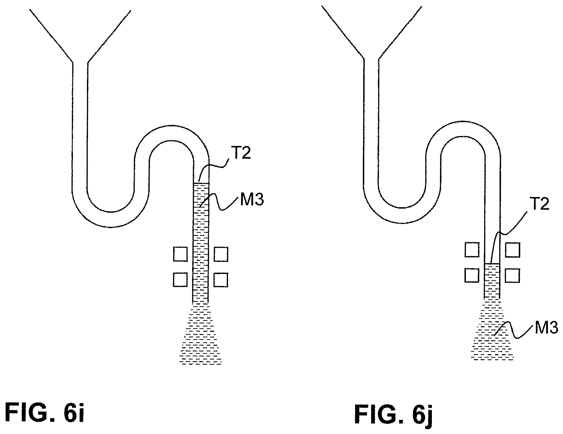

In den Figuren 6a bis 6j sind dieser Milchkanalabschnitt 36, der Einlass 35 mit dem eingangsseitigen Trichter mit der Schrägfläche 302 und die zwei Lichtschranken 6, 7 nochmals schematisch dargestellt. Das Grundprinzip der Messungen mittels des Siphons wird anhand dieser Zeichnungen erläutert.

In Figur 6a gelangt abgepumpte Milch M1 in den Einlass 35. In den Figuren 6b und 6c folgt weitere abgepumpte Milch M1 tropfenweise nach und gelangt so in den Milchkanalabschnitt 36, genauer in den ersten Kanalbereich 360. Der erste Siphonbogen 361 bildet einen Staubereich, wo sich die Milch zu einem Milchsee M2 sammelt. Luft aus dem Milch-Luft-Gemisch gelangt dabei bereits in den nächsten Bereich des Milchkanalabschnitts 36 und wird über den Auslass 37 weggeführt. Dies ist in Figur 6d erkennbar. Der Milchsee M2 wird nachfolgend immer grösser, wie in Figur 1e dargestellt ist. Er steigt dem zweiten Kanalbereich 362 entlang hoch, bis er den oberen Siphonbogen 363 erreicht und dessen Zenit passiert. Dies löst nun den Siphoneffekt aus, d.h. die angestaute Milch bewegt sich als Milchpaket M3, auch Milchvolumen oder Milchblock genannt, in Richtung Auslass 37. Dies ist in den Figuren 6g bis 6j gut erkennbar. Die währenddessen abgepumpte Milch M1 gelangt wiederum lediglich bis zum ersten Siphonbogen 361, wo sich wiederum ein Milchsee M2 zu einem Milchpaket M3 vergrössert.

Das Milchpaket M3 bildet zwei klare Trennungslinien T1, T2 zwischen Luft und Milch aus. Diese Trennungslinien T1, T2 lassen sich mittels Sensoren, beispielsweise mittels optischer Sensoren und insbesondere mittels der hier dargestellten Lichtschranken 6, 7, sehr genau detektieren. Die dadurch erhaltenen Signale lassen sich mittels des Signalauswertemoduls der Elektronik verarbeiten und es lassen sich diverse Messungen durchführen.

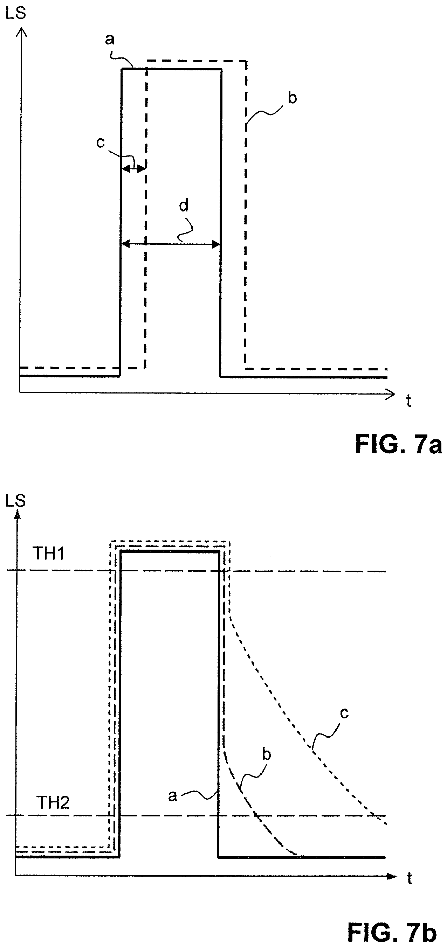

In Figur 7a ist beispielsweise aufgezeigt, wie mittels der zwei in Strömungsrichtung hintereinander angeordneten Lichtschranken 6, 7 die Geschwindigkeit des Milchdurchflusses und anschliessend das Milchvolumen bestimmt werden kann. Das Bezugszeichen a bezeichnet das Signal LS der ersten Lichtschranke 6, das Bezugszeichen b das Signal LS der zweiten Lichtschranke 7 in Bezug zur Zeit t. d bezeichnet somit die Zeitdauer, welche das Milchpaket M3 zum Durchlaufen einer der zwei Lichtschranken 6, 7 benötigt. c bezeichnet die Zeitverschiebung des Signals, da ja das Milchpaket M3 zuerst die erste Lichtschranke 6 und erst zeitverzögert die zweite Lichtschranke 7 passiert.

Die Geschwindigkeit $v_i$ eines Pakets ist dann:

$$v_i = (\text{Abstand zwischen den zwei Lichtschranken}) : c_i$$

Das Volumen V ergibt sich aus:

$$V_i = v_i * (\text{Querschnittsfläche des Milchkanalabschnitts}) * d_i$$

Das Gesamtvolumen V, d.h. die gesamte Menge abgepumpter Muttermilch ergibt sich aus:

$$V_{total} = \Sigma\ V_i$$

In Figur 7b ist als weiteres Beispiel die Bestimmung des Makronährwerts dargestellt. Dieser dient auch als Indikator für den Fettgehalt der Milch, da die Viskosität, d.h. das Fliessverhalten der Milch, wesentlich vom Fettgehalt beeinflusst ist.
In Figur 7b ist der Signalverlauf einer einzelnen Lichtschranke, z.B. der ersten Lichtschranke 6 dargestellt, wobei ein erster und ein zweiter Schwellwert TH1, TH2 des Lichtschrankensignals zur weiteren Auswertung verwendet werden. Die Kurve a zeigt ein Milchpaket M3 mit einem tiefen Makronährwert, die Kurve b ein Milchpaket M3 mit einem mittleren Makronährwert und die Kurve c ein Milchpaket M3 mit einem hohen Makronährwert. Dies ist anhand des sich verschlechternden Fliessverhaltens der Milch zu erkennen.

Ferner lassen sich die Anzahl der während einer Zeitspanne durchgeströmten Milchpakete M3 bestimmen. Es lassen sich auch die Frequenz des Durchfliessens der Milchpakete bestimmen. Ferner sind andere Methoden möglich, um die Viskosität der Milchpakete und somit den Makronährwert zu bestimmen.

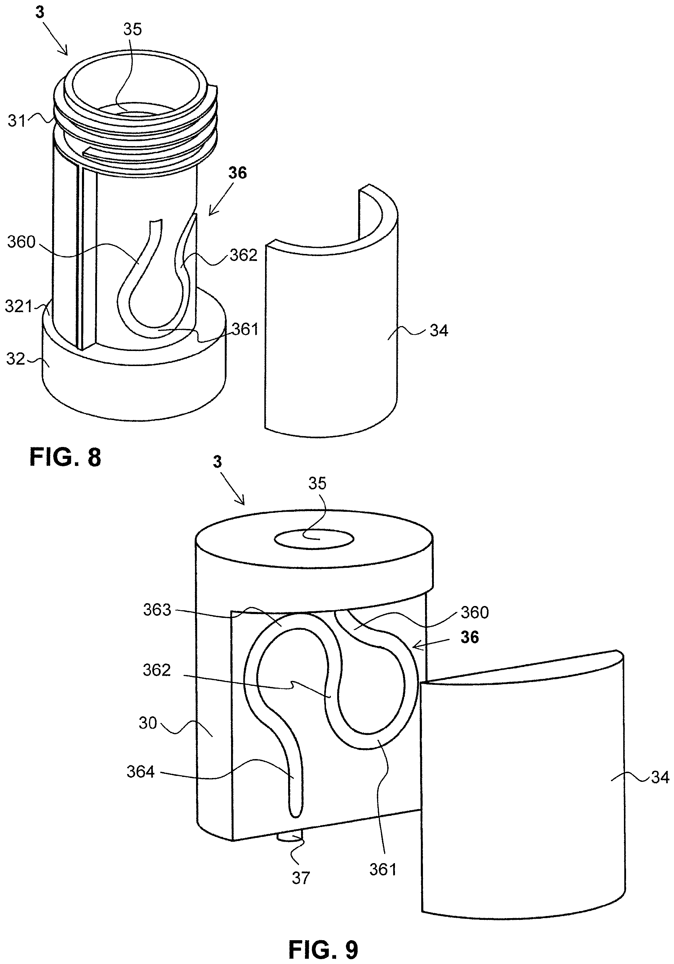

In den Figuren 8 bis 14 sind weitere Beispiele von erfindungsgemässen Sensoreinheiten 3 dargestellt, welche sich wie die bereits beschriebene Sensoreinheit 3 in der beschriebenen Brusthaubeneinheit oder in anderen Bereichen der Brustpumpe verwenden lässt. Der Milchkanalabschnitt 63 weist jeweils dieselben Kanalbereiche und Siphonbögen auf wie im ersten Beispiel, auch wenn in den Figuren nicht alle diese Elemente sichtbar sind.

Im Ausführungsbeispiel gemäss Figur 8 ist der Grundkörper 30 der Sensoreinheit 3 im Wesentlichen kreiszylinderförmig ausgebildet. Die Abdeckung 34 des Milchkanalabschnitts 36 ist als Halbschale ausgebildet. Das untere Anschlussteil 32 weist einen nach oben gerichteten Absatz 321 auf, auf welchem die Abdeckung 34 aufliegt.

In der Ausführungsform gemäss Figur 9 weist der Einlass 35 einen kleineren Durchmesser auf als in den vorherigen Beispielen. Der als Nut ausgebildete Teil des Milchkanalabschnitts 36 ist in einer planen Fläche und nicht wie in den vorherigen Beispielen auf einem gebogenen Mantelbereich ausgebildet. Die Abdeckung 34 weist entsprechend einen segmentförmigen Querschnitt auf und ist ebenfalls zum Grundkörper 30 hin plan ausgebildet.

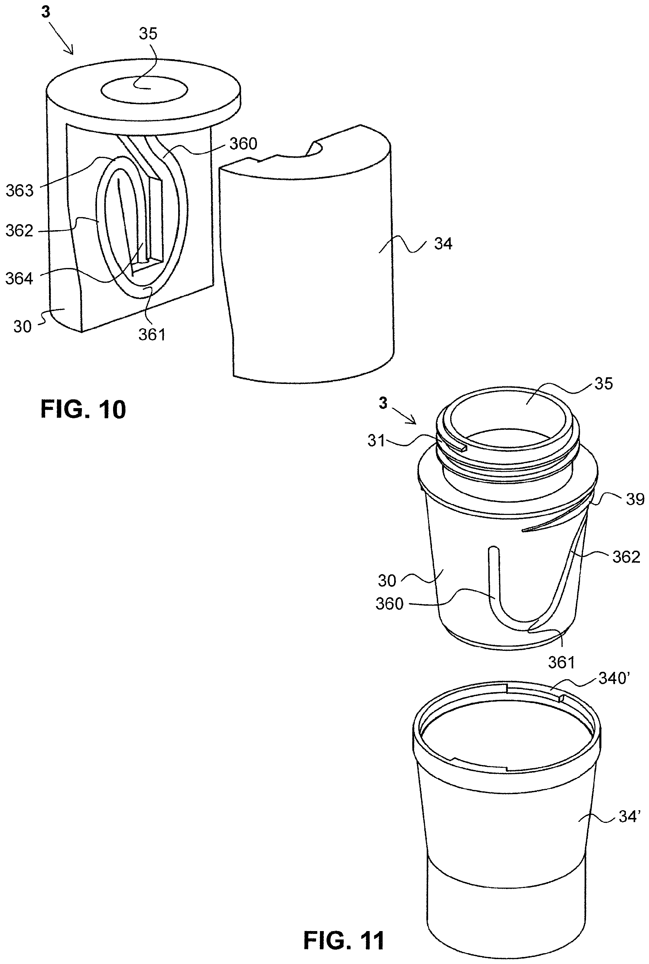

Im Ausführungsbeispiel gemäss Figur 10 sind der Grundkörper 30 und die Abdeckung 34 mit Stufen versehen. Der Siphon ist so ausgebildet, dass der obere Siphonbogen 363 mindestens teilweise senkrecht über dem unteren Siphonbogen 361 verläuft.

In Figur 11 ist die Abdeckung 34' als Hülse ausgebildet, welche über den Grundkörper 10 gestülpt wird, wobei die zwei Bauteile mittels Verschlusselemente 340', 39 ineinander rasten.

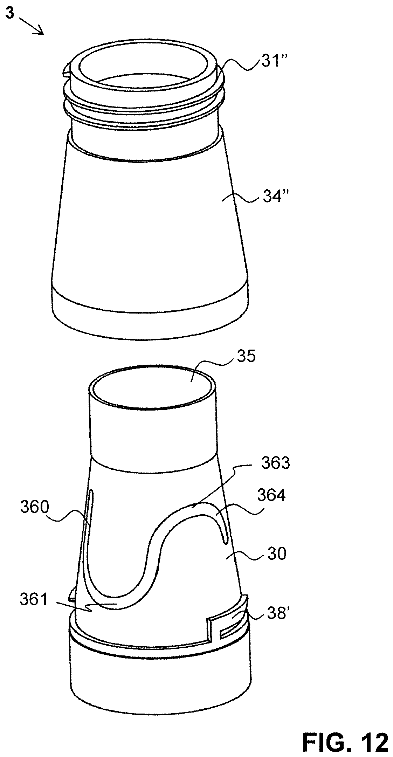

Figur 12 zeigt die eingangs anhand der Figuren 1 bis 5 beschriebene erste Ausführungsform. Diese Brusthaubeneinheit weist eine Hülse als Abdeckung 34" auf, welche im Gegensatz zum vorher beschriebenen Beispiel von oben über den Grundkörper 30 gestülpt wird.

In diesen Beispielen ist die Nut des Milchkanalabschnitts 36 stets auf der äusseren Oberfläche des Grundkörpers angeordnet. Sie kann zusätzlich oder alternativ auch in der Abdeckung angeordnet sein.

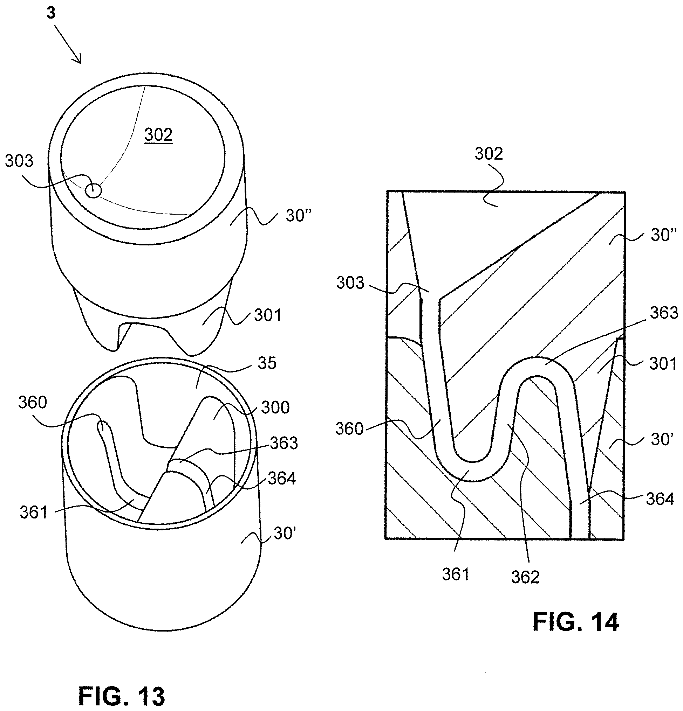

Im Ausführungsbeispiel gemäss den Figuren 13 und 14 ist der Milchkanalabschnitt 36 vollständig im Innern der Sensoreinheit 3 ausgebildet. Ein unterer Grundkörper 30' weist eine untere innere Wellenstruktur 300 auf, entlang deren Oberfläche die nach oben offene Nut des Milchkanalabschnitts 36 verläuft. Ein oberer Grundkörper 30" dient als Abdeckung und weist eine obere Wellenstruktur 301 auf, welche das Gegenstück zur unteren Wellenstruktur 300 bildet. Zudem verfügt der obere Grundkörper 30" vorzugsweise über einen Einlass mit einer Schrägfläche 302 und einer azentrischen Einlassöffnung 303, welche den Zugang zum siphonartigen Milchkanalabschnitt 36 bildet. In Figur 14 sind der obere und der untere Grundkörper 30", 30' im zusammengesetzten Zustand dargestellt, wodurch der Milchkanalabschnitt wiederum bis auf seinen Einlass und seinen Auslass geschlossen ausgebildet ist.

All diese Ausführungsbeispiele ermöglichen eine optimale Platzierung der Sensoren und der Elektronikeinheit und ermöglichen dank des nutartigen Siphons und der entfernbaren Abdeckung eine einfache und gründliche Reinigung.

Die erfindungsgemässe Brusthaubeneinheit ermöglicht eine optimale Messung von Eigenschaften der abgepumpten Muttermilch.

## BEZUGSZEICHENLISTE

| | | | | |
|---|---|---|---|---|
| 1 | Brusthaube | | 360 | erster Kanalbereich |
| 10 | Schild | | 361 | unterer Siphonbogen |
| 11 | Stutzen | | 362 | zweiter Kanalbereich |
| 12 | Abpumpbereich | | 363 | oberer Siphonbogen |
| | | | 364 | dritter Kanalbereich |
| 2 | Brusthaubenadapter | | 365 | vierter Kanalbereich |
| 20 | Grundkörper | | 366 | Dichtlippe |
| 21 | Anschlussstutzen | | 37 | Auslass |
| 22 | Ventil | | 38 | Hohlraum |
| | | | 39 | zweites Verschlusselement |
| 3 | Sensoreinheit | | 39' | Verschlusselement |
| 30 | Grundkörper | | | |
| 30' | unterer Grundkörper | | 4 | Milchsammelbehälter |
| 30" | oberer Grundkörper | | 40 | Grundkörper |
| 300 | untere Wellenstruktur | | 41 | Hals |
| 301 | obere Wellenstruktur | | | |
| 302 | Schrägfläche | | 5 | Elektronikeinheit |
| 303 | Einlassöffnung | | 50 | Batterie |
| 304 | Auslassöffnung | | 51 | Reedschalter |
| 31 | oberes Anschlussteil | | 52 | Mikrocontroller |
| 31' | oberes Anschlussteil | | 53 | Leiterplatte |
| 32 | unteres Anschlussteil | | 54 | Beschleunigungssensor |
| 320 | Innengewinde | | | |

| 321 | Absatz | 6 | erste Lichtschranke |
| 33 | Halteelement | | |
| 34 | Abdeckung | 7 | zweite Lichtschranke |
| 34' | untere Hülse | | |
| 34" 340' | obere Hülse | M1 | abgepumpte Milch |
| 340' | erstes Verschlusselement | M2 | Milchsee |
| 35 | Einlass | M3 | Milchpaket |
| 36 | Milchkanalabschnitt | | |
| T1 | erste Trennungslinie | | |
| T2 | zweite Trennungslinie | | |

**Patentansprüche**

1. Brusthaubeneinheit einer Brustpumpe zum Abpumpen von menschlicher Muttermilch, wobei die Brusthaubeneinheit eine Brusthaube (1) zur Auflage an eine Mutterbrust, einen Milchsammelbehälter (4) zur Aufnahme von abgepumpter Muttermilch, einen Brusthaubenadapter (2) zur Verbindung der Brusthaube (1) mit dem Milchsammelbehälter (4) sowie mindestens einen Sensor (6, 7) zur Detektion der Muttermilch aufweist, wobei sich ein Milchkanal von der Brusthaube durch den Brusthaubenadapter in den Milchsammelbehälter erstreckt, wobei der Milchkanal eine Strömungsrichtung der Muttermilch definiert, **dadurch gekennzeichnet, dass** im Milchkanal ein Siphon angeordnet ist und dass der mindestens eine Sensor (6, 7) zur Detektion der Muttermilch in Strömungsrichtung der Muttermilch nach dem Siphon angeordnet ist.

2. Brusthaubeneinheit nach Anspruch 1, wobei ein Ventil (22) vorhanden ist, welches den Milchkanal während des Abpumpens schliesst und öffnet und wobei der Siphon im Milchkanal in Strömungsrichtung der Muttermilch nach dem Ventil (22) angeordnet ist.

3. Brusthaubeneinheit nach Anspruch 2, wobei der Milchkanal zwischen Ventil (22) und Siphon eine Erweiterung aufweist.

4. Brusthaubeneinheit nach einem der Ansprüche 1 bis 3, wobei sie ein Signalauswertemodul zur Auswertung von Signalen des mindestens einen Sensors (6, 7) aufweist, wobei das Signalauswertemodul einen Verlauf der Signale in Funktion der Zeit liefert, wodurch mindestens eine der folgenden Grössen bestimmbar ist: Durchflussmenge, Durchflussrate, Durchflussgeschwindigkeit, Makronährstoffe, Fettgehalt.

5. Brusthaubeneinheit nach einem der Ansprüche 1 bis 4, wobei eine Sensoreinheit (3) vorhanden ist, welche vorzugsweise zwischen dem Brusthaubenadapter (2) und dem Milchsammelbehälter (4) angeordnet ist, wobei die Sensoreinheit (3) den Siphon des Milchkanals und den mindestens einen Sensor (6, 7) aufweist.

6. Brusthaubeneinheit nach Anspruch 5, wobei die Sensoreinheit (3) einen Grundkörper (30, 30', 30") aufweist mit einer Oberfläche und einer diese Oberfläche dicht verschliessenden Abdeckung (43, 43'), wobei mindestens der Siphon des Milchkanals durch eine Nut gebildet ist, welche in der Oberfläche und/oder in der Abdeckung ausgebildet ist und welche durch das Zusammenwirken der Oberfläche mit der Abdeckung bis auf einen Einlass (35) und einen Auslass (37) dicht verschlossen ist.

7. Brusthaubeneinheit nach Anspruch 6, wobei die Oberfläche eine radial nach aussen gerichtete Oberfläche des Grundkörpers (30) ist.

8. Brusthaubeneinheit nach Anspruch 7, wobei die Abdeckung (34, 34') ein Deckel oder eine Hülse ist.

9. Brusthaubeneinheit nach Anspruch 6, wobei die Sensoreinheit (3) einen nach oben offenen Innenraum aufweist, in welchem die Oberfläche in einer Wellenform (300) ausgebildet ist, und wobei die Abdeckung als wellenförmiges Gegenstück ausgebildet ist.

10. Brusthaubeneinheit nach einem der Ansprüche 6 bis 9, wobei der Grundkörper (30, 30', 30") einen Hohlraum zur Aufnahme einer Elektronikeinheit (5) aufweist.

11. Brusthaubeneinheit nach Anspruch 10, wobei die Elektronikeinheit (5) das Signalauswertemodul aufweist.

12. Brusthaubeneinheit nach einem der Ansprüche 5 bis 11, wobei die Sensoreinheit (3) kreiszylindeiförmig oder kegelstumpfförmig ausgebildet ist.

13. Brusthaubeneinheit nach einem der Ansprüche 1 bis 12, wobei der mindestens eine Sensor (6, 7) mindestens ein optischer Sensor ist.

14. Brusthaubeneinheit nach einem der Ansprüche 1 bis 13, wobei mehr als ein Sensor (6, 7) vorhanden ist und wobei unterschiedliche Sensoren (6, 7) vorhanden sind.

15. Sensoreinheit einer Brustpumpe zum Abpumpen von menschlicher Muttermilch, wobei die Brustpumpe eine Brusthaube (1) zur Auflage an eine Mutterbrust, einen Milchsammelbehälter (4) zur Aufnahme von abgepumpter Muttermilch, einen Milchkanal, welcher sich von der Brusthaube (1) zum Milchsammelbehälter (4) erstreckt, sowie mindestens einen Sensor (6, 7) zur Detektion der Muttermilch aufweist, wobei der Milchkanal eine Strömungsrichtung der Muttermilch definiert, **dadurch gekennzeichnet, dass** die Sensoreinheit (3) einen Teil des Milchkanals aufweist, wobei dieser Teil des Milchkanals einen Siphon ausbildet, und dass der mindestens eine Sensor (6, 7) zur Detektion der Muttermilch in der Sensoreinheit (3) in Strömungsrichtung der Muttermilch nach dem Siphon angeordnet ist.

16. Verfahren zur Bestimmung mindestens einer Eigenschaft von menschlicher Muttermilch während eines Abpumpens der menschlichen Muttermilch, wobei die Muttermilch durch Anlegen eines sich ändernden Unterdrucks abgepumpt wird, wobei das Verfahren folgende Schritte aufweist:

- Sammeln der Muttermilch in einem Staubereich eines Siphonbereichs,
- blockweises Entleeren des Staubereichs über eine Siphonschleife des Siphonbereichs unter Bildung eines Muttermilchpakets,
- Weiterleiten des Muttermilchpakets zu mindestens einem Sensor,
- Detektion des blockweise weitergeleiteten Muttermilchpakets mittels des mindestens eines Sensors und
- Auswertung von Signalen des Sensors in Funktion der Zeit.

**FIG. 1**

**FIG. 2**

FIG. 3

FIG. 4

FIG. 5

**FIG. 6a**

**FIG. 6b**

**FIG. 6c**

**FIG. 6d**

M1

M2

**FIG. 6e**

M1

M3

**FIG. 6f**

M3

M1

T2

T1

**FIG. 6g**

M3

M1

T2

T1

**FIG. 6h**

**FIG. 6i**

**FIG. 6j**

**FIG. 7a**

**FIG. 7b**

FIG. 8

FIG. 9

**FIG. 10**

**FIG. 11**

FIG. 12

**3**

302

303

30"

301

35

360

300

363

364

361

30'

**FIG. 13**

302

30"

303

363

301

360

30'

364

361    362

**FIG. 14**

**Europäisches Patentamt**
**European Patent Office**
**Office européen des brevets**

# EUROPÄISCHER RECHERCHENBERICHT

Nummer der Anmeldung

EP 18 16 0184

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y,D | US 2016/220743 A1 (GUTHRIE GABRIELLE V [US] ET AL) 4. August 2016 (2016-08-04) | 1-5, 12-15 | INV. A61M1/06 |
| A | * Absätze [0049] - [0071]; Anspruch 1; Abbildungen 5-12 * | 6-11 | G01W1/14 |
| | ----- | | |
| Y,D | DE 41 06 995 A1 (DURANGO HOLDING GMBH [DE]) 24. September 1992 (1992-09-24) | 1-5, 12-15 | |
| A | * Sätze 6-55; Abbildungen 1, 2 * <br> * Zusammenfassung * | 6-11 | |
| | ----- | | |
| A | US 361 910 A (B. TUTTON) 26. April 1887 (1887-04-26) * Seite 1, Zeile 29 - Seite 2, Zeile 17; Abbildungen 1, 2 * | 1,15 | |
| | ----- | | |
| A | WO 2016/014469 A1 (EXPLORAMED NC7 LLC [US]) 28. Januar 2016 (2016-01-28) * Absätze [0162] - [0174]; Abbildungen 1, 6-9 * | 1,15 | |
| | ----- | | **RECHERCHIERTE SACHGEBIETE (IPC)** <br><br> A61M <br> G01W |

Der vorliegende Recherchenbericht wurde für alle Patentansprüche erstellt

| Recherchenort | Abschlußdatum der Recherche | Prüfer |
|---|---|---|
| Den Haag | 1. August 2018 | Schlaug, Martin |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 16 0184

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-08-2018

| Im Recherchenbericht angeführtes Patentdokument | Datum der Veröffentlichung | Mitglied(er) der Patentfamilie | Datum der Veröffentlichung |
|---|---|---|---|
| US 2016220743 A1 | 04-08-2016 | US 2016220743 A1<br>US 2017220753 A1 | 04-08-2016<br>03-08-2017 |
| DE 4106995 A1 | 24-09-1992 | AT 132721 T<br>AU 1343992 A<br>DE 4106995 A1<br>DE 59205026 D1<br>DK 0574480 T3<br>EP 0574480 A1<br>ES 2084352 T3<br>JP 2599875 B2<br>JP H06504698 A<br>US 5483830 A<br>WO 9215249 A1 | 15-01-1996<br>06-10-1992<br>24-09-1992<br>22-02-1996<br>20-05-1996<br>22-12-1993<br>01-05-1996<br>16-04-1997<br>02-06-1994<br>16-01-1996<br>17-09-1992 |
| US 361910 A | 26-04-1887 | KEINE | |
| WO 2016014469 A1 | 28-01-2016 | AU 2015292839 A1<br>CA 2955939 A1<br>CN 107182202 A<br>EP 3171907 A1<br>JP 2017527337 A<br>KR 20170044650 A<br>SG 11201700489W A<br>US 2016206794 A1<br>US 2016287769 A1<br>US 2016303298 A1<br>US 2016310649 A1<br>US 2016310650 A1<br>US 2018001002 A1<br>WO 2016014469 A1 | 16-02-2017<br>28-01-2016<br>19-09-2017<br>31-05-2017<br>21-09-2017<br>25-04-2017<br>27-02-2017<br>21-07-2016<br>06-10-2016<br>20-10-2016<br>27-10-2016<br>27-10-2016<br>04-01-2018<br>28-01-2016 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014161099 A1
- US 20160220743 A1
- US 20150283311 A1
- US 20170021068 A1
- DE 4106995 A1
- DE 4114933 A1

- US 3641818 A
- US 3919455 A
- US 5656027 A
- GB 2031158 A
- WO 2010149708 A1